# EUROPEAN PATENT SPECIFICATION

(11) **EP 2 967 793 B1**
(45) Date of publication and mention of the grant of the patent: **12.07.2017**
(21) Application number: 14715759.8
(22) Date of filing: 10.03.2014
(51) Int. Cl.: A61F 2/00

(54) **MEDICAL DEVICE FOR INJECTING A FLUID**
MEDIZINISCHE VORRICHTUNG ZUM EINSPRITZEN EINER FLÜSSIGKEIT
DISPOSITIF MÉDICAL D'INJECTION D'UN FLUIDE

(30) Priority: 11.03.2013 US 201361776428 P; 06.03.2014 US 201414199655
(43) Date of publication of application: 20.01.2016
(73) Proprietor: Boston Scientific Scimed, Inc., Maple Grove, MN 55311-1566 (US)
(72) Inventor: CHU, Michael S.H., Brookline, Massachusetts 02446 (US)
(74) Representative: Pfenning, Meinig & Partner mbB
(86) International application number: PCT/US2014/022509
(87) International publication number: WO 2014/164471

(56) References cited:
- US-A1- 2009 177 241
- US-A1- 2011 230 704
- US-A1- 2012 029 275
- US-A1- 2012 232 573

## Description

### CROSS REFERENCE TO RELATED APPLICATIONS

This application claims priority to, and is a continuation of, U.S. Nonprovisional Patent Application No. 14/199,655, filed on March 6, 2014, entitled "MEDICAL DEVICE AND METHOD FOR INJECTING A FLUID", which, in turn, claims priority to U.S. Provisional Patent Application No. 61/776,428, filed on March 11, 2013, entitled "MEDICAL DEVICE AND METHOD FOR INJECTING A FLUID".

This application also claims priority to U.S. Provisional Patent Application No. 61/776,428, filed on March 11, 2013.

### FIELD

The present invention generally relates to medical devices and procedures, and particularly to devices and methods for delivery of implants and medication in a patient's body.

### DESCRIPTION OF THE RELATED ART

Pelvic organ prolapse is an abnormal descent or herniation of the pelvic organs. A prolapse may occur when muscles and tissues in the pelvic region become weak and can no longer hold the pelvic organs in place correctly. This decrease in structural integrity of anatomical tissues may have significant medical consequences, which in turn might influence the biological functions of the tissues.

Treatment for symptoms of the pelvic organ prolapse can include changes in diet, weight control, and lifestyle. Treatment may also include surgery, medication, and use of grafts or implants to support the pelvic organs. For example, an implant can be placed in a patient to provide support for the weakened or damaged tissue. The implant may try to replicate the natural position and structure of, or otherwise, provide support to the tissue and thereby help in decreasing or eliminating impairment of biological functions resulting from tissue weakening or damage.

These surgical methods may use a delivery device for assisting delivery of the implant to the anatomical tissue inside the patient's body. A medication may be applied through the delivery device simultaneously during the introduction of the implant inside the body of the patient. The medication may be beneficial to a patient during placement of the implant, to such as manage post-surgical pain, or prevent infection or excess bleeding and the like. There may generally be a requirement of maintaining a tactile feedback with a needle of the delivery device during implant placement so as to prevent injury to any bodily tissues like urinary bladder. Therefore, the injection of the medication along with delivery of the implant may require alternative and frequent steps of stopping, aspirating and then injecting along the needle track while maintaining the tactile feedback. This may complicate the method of implant and medication delivery and also may be time consuming.

US 2011 / 0230704 A1 discloses a surgical assembly for treating incontinence including an elongate needle, a sling and a coupler, wherein the coupler has an elongate body with an axis and is convenient and easy to connect to the needle.

In view of the above, there is a need for a device and a surgical procedure that facilitates in delivery and placement of the implant and delivery of the medication or any other fluid inside the body.

### SUMMARY

The invention relates to a medical assembly as defined by the claims. Insofar as the terms "invention" is used in the following, and/or features are presented as being optional, this should be interpreted in such a way that the only protection sought is that of the invention as claimed. Methods of using the device are presented as a way to understand the invention and do not form part of the invention. The medical assembly comprises an implant assembly and an adaptor. The implant assembly includes a proximal portion and a distal portion. The adaptor can be configured to be coupled to the implant assembly at the distal portion of the implant assembly after at least a portion of the implant assembly extends through and out of the body. The adaptor further includes a proximal portion, a distal portion, and a lumen defined between the proximal and the distal portion. The adaptor further includes a locking mechanism for locking the adaptor to the distal portion of the implant assembly. The adaptor further includes a hub portion defined at the distal portion of the adaptor.

In an example not part of the invention it is disclosed a method for treatment of a pelvic floor disorder. The method includes guiding an implant assembly including an implant along a first direction from a first incision toward a second incision in a body of a patient such that at least a portion of the implant assembly protrudes out from the body of the patient through the second incision. The method further includes coupling a fluid delivery device to a portion of the implant assembly that protrudes out of the body through the second incision such that at least a portion of the fluid delivery device is outside of the body of the patient. The method further includes injecting a fluid using the fluid delivery device into the implant assembly in a direction that is different from the first direction.

In another example not part of the invention it is disclosed a method for treatment of a pelvic floor disorder. The method comprises guiding an implant assembly, including an implant, along a first direction from a first incision toward a second incision in a body of a patient. The implant is guided such that at least a portion of the implant assembly protrudes out from the body of the patient through the second incision. The method further includes coupling a proximal portion of an adaptor to the portion of the implant assembly that protrudes out through the second incision. The method further includes injecting a fluid through a lumen of the adaptor into the implant assembly in a direction that is different from the first direction.

### BRIEF DESCRIPTION OF THE FIGURES

The invention and the following detailed description of certain embodiments, thereof, may be understood with reference to the following figures:
FIG. 1 is a schematic diagram of a medical assembly, in accordance with an embodiment of the invention.
FIG. 2 is a perspective view of an implant assembly configured to be delivered and placed inside a patient's body, in accordance with an embodiment of the invention.
FIG. 3 is a perspective view of a delivery needle configured for delivering an implant assembly inside a patient's body, in accordance with an embodiment of the invention.
FIG. 4A is a perspective view of an adaptor configured for facilitating delivery of a fluid to bodily tissues inside the patient's body, in accordance with an embodiment of the invention.
FIG. 4B is a cross-sectional view of the adaptor of FIG. 4A.
FIG. 4C is a perspective view of an adaptor configured for facilitating delivery of a fluid to bodily tissues, in accordance with an embodiment of the invention.
FIG. 5 is a perspective view of a medical assembly for the treatment of a pelvic floor disorder, in accordance with an embodiment of the invention.
FIG. 6 is a perspective view of a spinal needle configured for injecting a drug inside a patient's body, in accordance with an embodiment.
FIG. 7A is a schematic view of a first incision and a second incision placed over a patient's body before insertion of the implant assembly of FIG. 2 inside the body, in accordance with an embodiment of the invention.
FIG. 7B is a perspective view of the implant assembly of FIG. 2 during insertion from the first incision toward the second incision inside a patient's body, in accordance with an embodiment of the invention.
FIG. 7C is a perspective view of the implant assembly of FIG. 2 while moving from the first incision and protruding out through the second incision inside a patient's body, in accordance with an embodiment of the invention.
FIG. 7D is a perspective view of the implant assembly of FIG. 2 and the adaptor ready to be coupled to the implant assembly after a portion of the implant assembly extends out of the patient's body from the second incision, in accordance with an embodiment of the invention.
FIG. 7E is a perspective view of the implant assembly of FIG. 2 and the adaptor coupled to the implant assembly that extends out of the patient's body, in accordance with an embodiment of the invention.
FIG. 8A is a schematic view of a first incision and a second incision placed over a patient's body before insertion of the implant assembly of FIG. 2 inside the body, in accordance with an embodiment of the invention.
FIG. 8B is a perspective view of a fluid delivery device coupled to a sleeve of the implant assembly, in accordance with an embodiment of the invention.
FIG. 8C is a perspective view of the fluid delivery device coupled to the sleeve and injecting a fluid into the implant assembly.
FIG. 8D is a perspective view of an end portion of a device in accordance with an embodiment of the invention.
FIG. 8E is a perspective view of an end portion of a device in accordance with an embodiment of the invention.
FIG. 9 is a flowchart illustrating a method of treatment of a pelvic floor disorder.

### DETAILED DESCRIPTION

Detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure. Further, the terms and phrases used herein are not intended to be limiting, but to provide an understandable description of the invention.

The terms "a" or "an," as used herein, are defined as one or more than one. The term "another," as used herein, is defined as at least a second or more. The terms "including" and/or "having", as used herein, are defined as comprising (i.e., open transition).

In general, the invention is directed to systems, methods, and devices for treating incontinence, such as urinary incontinence. However, the invention may be equally employed for other treatment purposes such as pelvic organ prolapse or other pelvic disorders. As described below in various illustrative embodiments, the invention provides systems, methods, and devices employing a medical device configured to deliver or place an implant within a patient's body to support pelvic organs and deliver a fluid such as a medication inside the body such as to the implant site for the treatment of incontinence or other pelvic disorders.

The term patient may be used hereafter for a person who benefits from the medical device or the methods disclosed in the present invention. For example, the patient may be a person whose body is operated with the use of the medical device disclosed by the present invention in a surgical treatment. For example, in some embodiments, the patient may be a human female, human male or any other mammal.

The terms proximal and distal described in relation to various devices, apparatuses, and components as discussed in the subsequent text of the present invention are referred to with a point of reference. The point of reference, as used in this description, is a perspective of an operator. The operator may be a surgeon, a physician, a nurse, a doctor, a technician, and the like who may perform the procedure of delivery and placement of the bodily implants into the patient's body as described in the present invention. The term proximal refers to an area that is closest to the operator. The term distal refers to an area that is farthest from the operator.

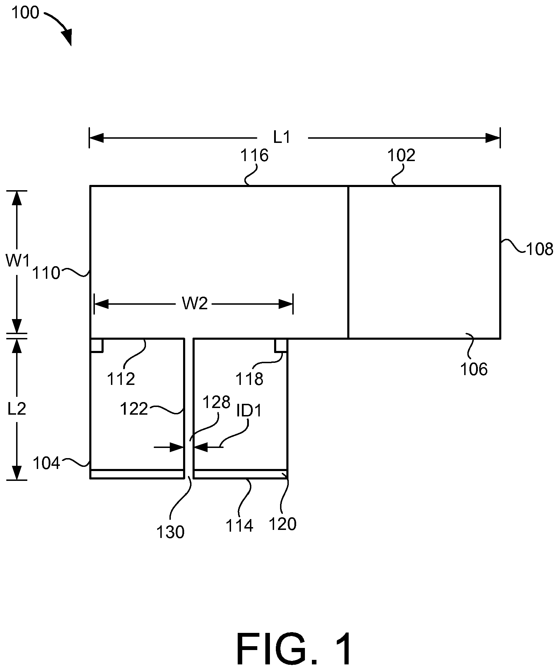

FIG. 1 is a schematic diagram of a medical assembly 100 in accordance with an embodiment of the present invention. The medical assembly 100 includes an implant assembly 102 and an adaptor 104. The implant assembly 102 includes an implant 106. In some embodiments, the implant assembly 102 can further include other elements, devices or components such as a dilator, a sleeve, and the like (not shown). In some embodiments, the implant assembly 102 can be used to suspend various bodily locations in a body of a patient. For example, in some embodiments, the implant assembly can be used to suspend a pelvic organ of a patient's body. In some embodiments, the implant assembly 102 can be a retropubic incontinence sling. In some embodiments, the implant assembly 102 can be configured to be delivered by way of a transvaginal approach or a transobturator approach or vaginal pre-pubic approach or can be delivered through other approaches and positioned at various locations within a patient's body.

The implant assembly 102 includes a first end portion 108 and a second end portion 110 such that the implant assembly 102 can be advanced inside a body of a patient with the second end portion 110 piercing through bodily tissues. The first end portion 108 can also be passed into the body to pierce through bodily tissues. The implant assembly 102 can define a length L1 and a width W1. In some embodiments, the width W1 can be the same or remain constant from the first end portion 108 to the second end portion 110. In some embodiments, the width W1 can differ from the first end portion 108 to the second end portion 110. In some embodiments, the assembly 102 can be configured to be coupled to a delivery device (not shown) for delivery of the assembly 102 inside a patient's body. In various embodiments, the delivery device can be configured to deliver the implant 106 to a delivery site or place or fix an implant 106 to a bodily tissue. The assembly 102 can be inserted inside a patient's body from a first incision and at least a portion of the assembly 102 can be extended out of the patient's body through a second incision. In some embodiments, the first incision can be a vaginal incision. In some embodiments, the second incision can be an abdominal incision or a groin incision. The implant assembly 102 can be configured to be coupled to the adaptor 104. In some embodiments, the implant assembly 102 is configured to be placed within a body of a patient and configured to be disposed proximate a portion of the body of the patient to provide support to the portion of the body of the patient. In some embodiments, the implant assembly 102 can include a mesh portion. In other embodiments, the implant assembly 102 can include a portion that has a sheet of material, which may be porous or non-porous.

The adaptor 104 includes a proximal portion 112, and a distal portion 114 and a lumen 128 extending from the proximal portion 112 to the distal portion 114. The lumen 128 defines an internal diameter ID1. In some embodiments, the dimension of the internal diameter ID1 can be the same or remain constant from the proximal portion 112 to the distal portion 114 of the adaptor 104. In some embodiments, the dimension of the internal diameter ID1 can vary from the proximal portion 112 to the distal portion 114 of the adaptor 104. The adaptor 104 defines a length L2 and a width W2. In some embodiments, the width W2 of the adaptor 104 can vary from the proximal portion 112 to the distal portion 114.

In some embodiments, the adaptor 104 can be configured to couple to the second end portion 110 of the implant assembly 102 after a portion of the implant assembly 102 extends through and out of the body at the second incision. In some embodiments, the proximal portion 112 of the adaptor 104 can be coupled to the second end portion 110 of the implant assembly 102. In some embodiments, the proximal portion 112 of the adaptor 104 can be configured to be coupled to a portion of the assembly 102 such as a dilator, a sleeve or the implant 106 or any other component of the implant assembly 102 after at least a portion of the implant assembly 102 or any of its components extends through and out the patient's body. For example, in some embodiments, the dilator can extend out of the patient's body. In some embodiments, the sleeve can extend out of the patient's body. In some embodiments, the implant 106 can extend out of the patient's body. In some embodiments, any other component of the assembly 102 can extend out of the patient's body. In some embodiments, the proximal portion 112 of the adaptor 104 can be coupled to a portion of the implant assembly 102 that protrudes out through the second incision. In some embodiments, the proximal portion 112 and a medial portion 116 of the adaptor 104 can be coupled to the portion of the implant assembly 102 that protrudes out through the second incision. The adaptor 104 can couple to the implant assembly 102 such that at least a portion of the implant assembly 102 is outside the patient's body.

The adaptor 104 may be configured for coupling and decoupling with the implant assembly 102 such as with the use of a rotational force applied by the operator from externally relative to the adaptor 104. In some embodiments, the distal portion 114 of the adaptor 104 can be fabricated so as to facilitate application of the rotational force. In some embodiments, the proximal portion 112 of the adaptor 104 can be more rigid as compared to the distal portion 114 of the adaptor 104. In some embodiments, the proximal portion 112 of the adaptor 104 is coupled to the implant assembly 102. The more-rigid proximal portion 112 can facilitate coupling of the adaptor 104 to the implant assembly 102. The less-rigid distal portion 112 can be maneuvered so as to couple the adaptor 104 with the implant assembly 102.

The adaptor 104 may further include a locking mechanism 118 for locking the adaptor 104 to the second end portion 110 of the implant assembly 102. The locking mechanism 118 can be fabricated at the proximal portion 112 of the adaptor 104. In some embodiments, the locking mechanism 118 can be configured by providing a plurality of protrusions for coupling with the assembly 102. In some embodiments, the plurality of protrusions can be fabricated over the proximal portion 112 of the adaptor 104. In some embodiments, the plurality of protrusions can be fabricated over the proximal portion 112 and the medial portion 116 of the adaptor 104. In some embodiments, the plurality of protrusions can include barbs, for snap fitting into the assembly 102. The barbs can have a rectangular, triangular, or oval cross section. In some embodiments, the locking mechanism 118 can include a snap-fitting arrangement for coupling the adaptor 104 to the implant assembly 102. In some embodiments, the locking mechanism 118 can include a frictional fitting arrangement for coupling the adaptor 104 to the implant assembly 102. In some embodiments, the locking mechanism 118 can include a plurality of threads at the proximal portion of the adaptor 104. The plurality of threads can be configured to thread-ably engage the second end portion 110 of the implant assembly 102. In some embodiments, the locking mechanism 118 can include a first coupler for coupling it to a second coupler of the implant assembly 102. In some embodiments, the first coupler can be a male coupler and the second coupler can be a female coupler. In some embodiments, the first coupler can be a female coupler and the second coupler can be a male coupler. In other embodiments, various other types of locking arrangements may be provided to couple the adaptor 104 to the implant assembly 102 and retain the adaptor 104 and the implant assembly 102 in the coupled state. The above description provides a discussion about an arrangement for coupling the proximal portion 112 of the adaptor 104 to the second end portion 110 of the implant assembly 102. In a similar manner, the proximal portion 112 of the adaptor 104 can be coupled to the first end portion 108 of the implant assembly 102 to place the implant 106 at a contra lateral side.

The adaptor 104 further includes a hub portion 120. The hub portion 120 can be defined at the distal portion 114 of the adaptor 104. The hub portion 120 may include a coupler 130 and a handle (not shown and explained later). The coupler 130 can be configured for coupling the adaptor 104 to a separate device. The device can include such as a fluid delivery device for delivery of a fluid inside the assembly 102. In some embodiments, the device can be a biopsy needle configured to collect a sample from the bodily tissues. In some embodiments, the device can be a needle configured to collect fluid samples from the bodily tissues. In some embodiments, the coupler 130 can be a male coupler, a female coupler, a thread-able engagement, a snap fit mechanism, and/or a frictional fit mechanism. The handle can be configured to facilitate application of a rotational force on the adaptor 104 with respect to the implant assembly 102 for coupling and decoupling the implant assembly 102 and the adaptor 104.

In some embodiments, the adaptor 104 may include a tubular structure 122 defined at the proximal portion 112 of the adaptor 104. The tubular structure 122 can be configured to be inserted within the implant assembly 200. In some embodiments, the proximal portion 112 can be fabricated to have a tubular shape. A length of the tubular structure 122 can varied as per requirement.

In some embodiments, the adaptor 104 can be symmetrical at both the portions - proximal and distal portions 112 and 114. In such embodiments, for example, a first coupler and a second coupler similar to the coupler 130 discussed above can be provided on each of the distal and proximal portions such that the implant assembly or the fluid delivery device can be connected on either side. In other embodiments, the proximal portion 112 may include a coupler customized to suit the implantable assembly or a component thereof such as the dilator or the sleeve. In other embodiments, there may not be any coupler on the proximal portion 112 at all. The proximal portion 112 or a tubular shaped portion defined at the proximal portion 112 can itself be inserted inside the implant assembly 102 such as the dilator or the sleeve for fixing the adaptor 104 to the implant assembly 102 at least temporarily during the procedure. In some embodiments, the coupler provided at the distal portion 114 for coupling to an external device, may be custom shaped and designed to fit the requirements of the external device such as the fluid delivery device.

The implant assembly 102 can be guided such as along a first direction from the first incision toward the second incision in the patient's body such that at least a portion of the implant assembly 102 protrudes out from the body of the patient through the second incision.

The adaptor 104 can be configured for injection of a fluid through the lumen 128 of the adaptor 104 to a target site proximate the implant assembly 102 in a body of a patient such that a direction of the delivery of the fluid is different from a direction of the delivery of the implant assembly 102 inside the patient's body. For example, the implant assembly 102 can be inserted inside the patient's body from the vaginal incision and moved towards the abdominal incision. The implant assembly 102 may be advanced from the vaginal incision toward the abdominal incision in the first direction. A portion of the implant assembly 102 may extend out of the abdominal incision after the implant assembly 102 reaches the abdomen of the patient's body. The adaptor 104 can be coupled to the implant assembly 102 or any portion thereof to provide a fluid communication channel from the adaptor 104 to the implant assembly 102. As the adaptor 104 is coupled to the implant assembly 102 proximate the abdominal incision, the fluid injected through the adaptor 104 travels along a second direction from the abdominal incision to the vaginal incision. The first direction and the second direction can be substantially different from one other. In some embodiments, the first direction and the second direction can be opposite to each other. In some embodiments, the fluid injected through the adaptor 104 travels from the second incision to the first incision along a substantially curved path inside the patient's body. Similarly, the implant assembly 102 may be delivered from the first incision to the second incision along a curved path. In such cases, the direction that the fluid traces along the curved path during injection of the fluid is different from the direction of delivery of the implant assembly 102 along the curved path.

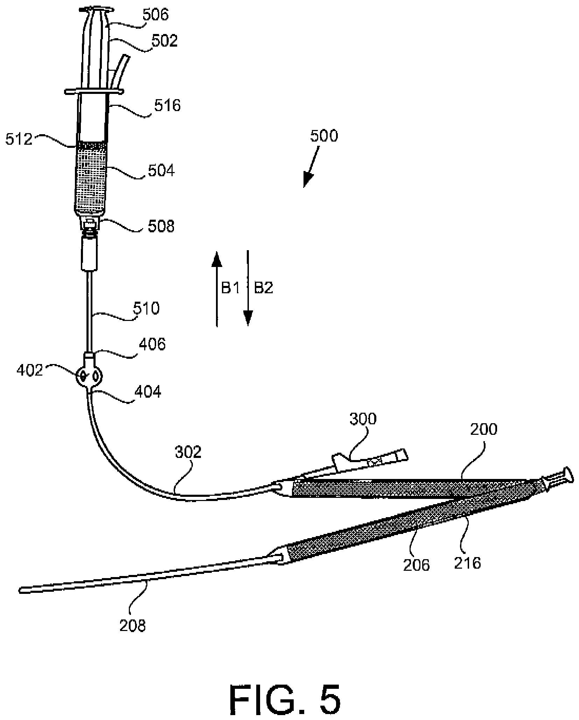

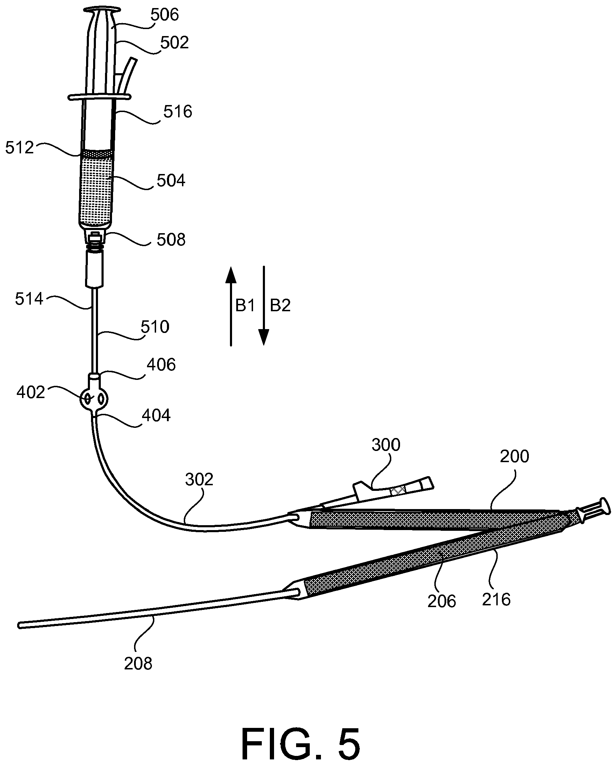

In some embodiments, the adaptor 104 may perform as a fluid injector and may directly be configured to deliver the fluid to the body tissues proximate the implant site without requiring any separate fluid injection or delivery device. In some other embodiments, a separate fluid delivery device (not shown here and explained later by way of FIG. 5) can be required to be coupled to the adaptor 104 for facilitating fluid delivery. The fluid delivery device, in such cases, can be a syringe, a plunger or any other similar device capable of injecting fluid. In some embodiments, the fluid can be a medication such as anesthetics or drugs and the like.

After at least a portion of the implant assembly 102 protrudes out of the second incision, the implant assembly 102 can be pulled along the first direction as the fluid is injected along the second direction. This pulling facilitates placing of the implant 106 at a delivery site inside the patient's body.

In some embodiments, an operator can aspirate along the assembly 102 so as to determine presence of any blood vessels that can be present along a track of the implant assembly 102. The portion of the implant assembly 102 that protrudes out through the second incision can be pulled along the first direction on detection of any blood vessels along the track of the implant assembly 102 to reposition the implant 106 to a location where such blood vessels are not identified.

After placing in the implant and delivering the fluid inside the patient's body, the portion of the implant assembly 102 that protrudes out through the second incision can be pulled out of the patients' body so as to decouple at least some components of the implant assembly 102 such as the sleeve or the dilator from the implant 106 after placing the implant 106.

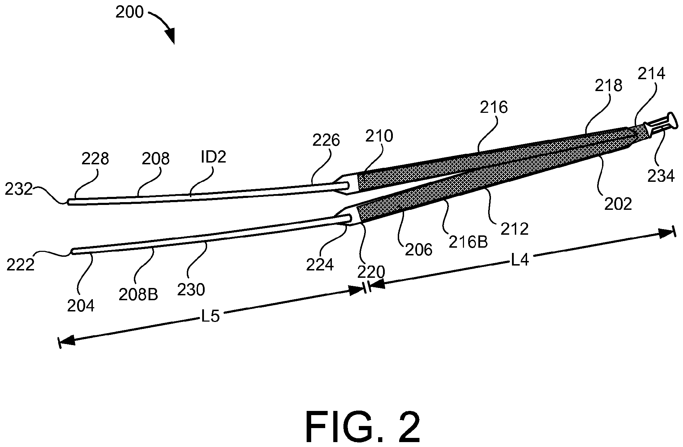

In accordance with various embodiments as discussed above, each of the first end portion 108 and the second end portion 110 of the implant assembly 102 are inserted inside the body one by one at contra lateral sides such that a portion of each of the first end portion 108 and the second end portion 110 of the implant assembly 102 protrudes outside abdominal incisions made at respective contra lateral sides. This facilitates placing of the two end portions of the implant at the respective contra lateral sides in a similar manner. FIG. 2 is a perspective view of an implant assembly 200. The implant assembly 200 can be configured to be delivered and placed inside a patient's body. The implant assembly 200 includes a first end portion 202 and a second end portion 204. The implant assembly 200 can be inserted inside the patient's body from such as a first incision and moved toward a second incision such that at least a portion of the implant assembly 200 protrudes out of the patient's body from the second incision. In some embodiments, the first incision can be a vaginal incision. In some embodiments, the second incision can be an abdominal or groin incision.

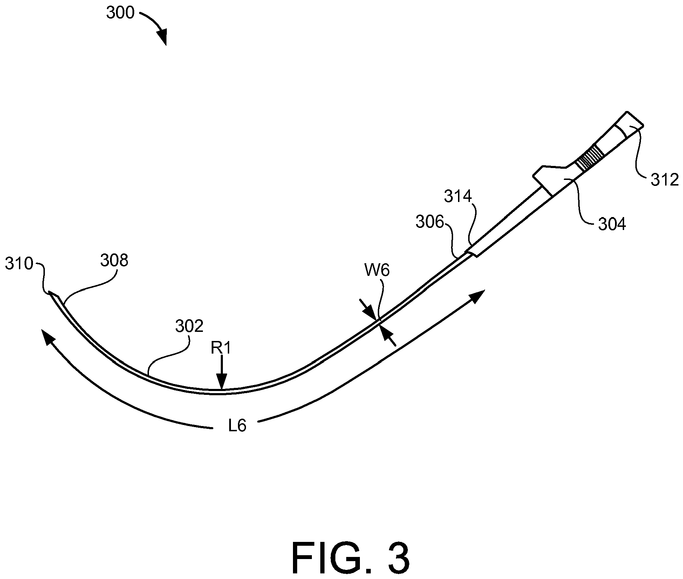

The implant assembly 200 can include an implant 206. The implant assembly 200 can include a dilator 208 coupled to such as at the second end portion 204. The dilator 208 can be coupled to the implant 206 such that an operator can maneuver the implant 206 for placing the implant 206 at a delivery site by maneuvering the dilator 208. In some embodiments, a delivery device or a delivery needle (explained later in FIG. 3) can be used for delivering the implant assembly 200 inside the patient's body.

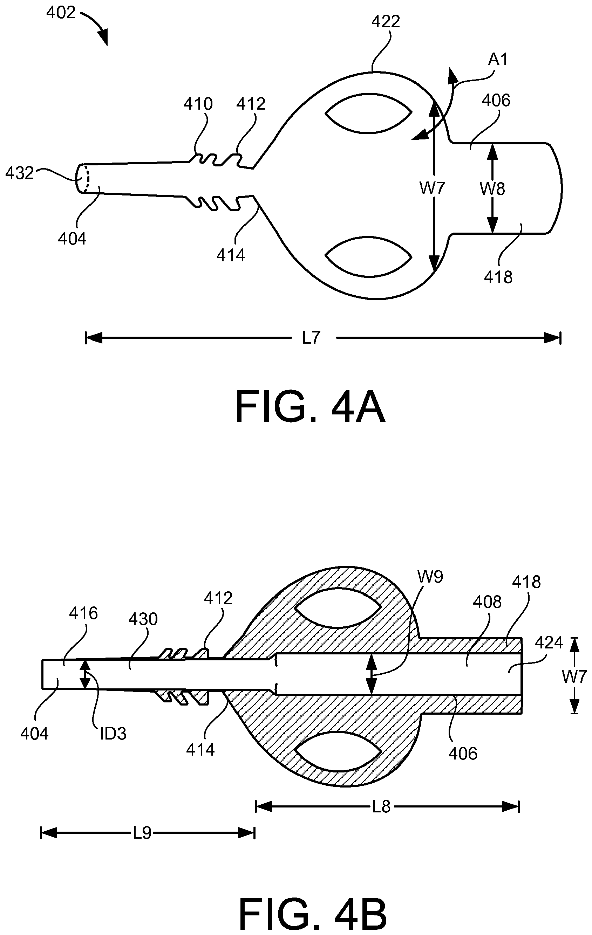

The implant 206 includes a first end portion 210 and a second end portion 212 at contra lateral sides. In some embodiments, each of the first and second end portions 210 and 212 can be maneuvered independent of each other. In some embodiments, the implant 206 can be inserted into the patient's body such that after placement, a central portion 214 of the implant 206 is proximate the first incision and the first end portion 210 is proximate the second incision. A plurality of incisions configured as the second incision can be made such that each of the first end portion 210 and the second end portion 212 are proximate to one of the incisions configured as the second incision. The implant 206 can be further coupled to a sleeve 216 which can cover at least a portion of the implant 206. The sleeve 216 can define a proximal portion 218 and a distal portion 220 such that the proximal portion 218 of the sleeve 216 is proximate the first incision and the distal portion 220 is proximate the second incision. The sleeve 216 can define a length L4 extending from the proximal portion 218 to the distal portion 220 of the sleeve 216. In some embodiments, the distal portion 220 of the sleeve 216 can be coupled to the dilator 208. In some embodiments, the distal portion 220 of the sleeve 216 or the dilator 208 can be configured to be coupled to an adaptor (explained later in FIGS. 4A-4C) after a portion of the implant assembly 200 extends out of the second incision.

The implant assembly 200 can further include an inlet port 222 proximate or at the second end portion 204. The inlet port 222 can be configured for receiving a fluid into the implant assembly 200. The implant assembly 200 includes an outlet port 224. The outlet port 224 can be in fluid communication with the inlet port 222. The outlet port 224 can be configured for receiving the fluid via the inlet port 222. The outlet port 224 can be located at the bodily tissues proximate the delivery site after the implant assembly 200 is inserted in the body. The inlet port 222 and the outlet port 224 can be so configured in a way that the fluid received into the implant assembly 200 from the inlet port 222 can be delivered to the bodily tissues proximate the delivery site through the outlet port 224. In some embodiments, the inlet port 222 can be fabricated on a portion of the dilator 208 and the outlet port 224 can be configured on a portion of the sleeve 216 or the implant 206. In some embodiments, the inlet port 222 may be configured at the distal portion 220 of the sleeve 216 or the implant 206, and the outlet port 224 can be configured toward the proximal portion 218 of the sleeve 216 or the implant 206.

The dilator 208 of the implant assembly 200 can be coupled to the implant 206 or the sleeve 216. The dilator 208 can be inserted from the first incision inside the patient's body. The dilator 208 can be configured and fabricated so that at least a portion of the dilator 208 can extend out of the patient's body at the second incision. The dilator 208 can be configured to be coupled to an adaptor (explained later in FIGS. 4A-4C). The dilator 208 defines a proximal portion 226 and a distal portion 228, and a lumen 230 extending from the proximal portion 226 to the distal portion 228. The dilator 208 defines a length L5 extending from the proximal portion 226 to the distal portion 228. The length L5 can vary based on requirements of the surgical process. In some embodiments, the distal portion 228 of the dilator 208 has a closed end tip portion 232. In some embodiments, the closed end tip 232 includes a needle connector. In some embodiments, the needle connector can be a loop fabricated into the distal portion 228 of the dilator 208. In some embodiments, the distal portion 228 can include a needle slot. In some embodiments, the needle slot can be L-shaped. In some embodiments, the needle slot can be T-shaped. The closed end tip portion 232 may be required to be cut for coupling the adaptor to the dilator 208. The lumen 230 of the dilator 208 defines an internal diameter ID2. The lumen 230 of the dilator 208 can be configured such as to receive a delivery needle (explained later in FIG. 3) for facilitating the delivery and placing of the implant 206 inside the patient's body. The dimension of the internal diameter ID2 can vary as per requirements and dimensions of the delivery needle. In some embodiments, the cross section of the dilator 208 can be circular, substantially flat or triangular in shape or can be of any other shape. In other embodiments, the cross section of the dilator 208 can be substantially rectangular and tapered at the distal portion 228 of the dilator 208. In some embodiments, the dilator 208 can be employed of any shape as per the requirements in a specific surgical procedure. In some embodiments, the dilator 208 can be made of a flexible material so as to be sized to assume the shape of the delivery needle on its insertion.

In some embodiments, the dilator 208 is a first dilator that can be attached to a first end portion 210 of the implant 206. In an embodiment, a second dilator 208b that can be similar to the first dilator 208 and can be attached to the second end portion 212 of the implant 206 may be provided. The dilator 208 can be configured to expand an opening in a patient's body allowing insertion of the implant 206 into the opening within a patient's body. In some embodiments, the proximal portion 226 of the dilator 208 can be extended distally to overlap with or partially enclose the implant 206.

In some embodiments, at least a portion of the implant assembly 200 can be made from one or more biocompatible materials such as a plastic or metal. In an example, at least a portion of the implant assembly 200 can be made of a semi-rigid plastic material. Examples of such materials include, but are not limited to, polyethylene terephthalate (PET), polyethylene (PE), or ethylene vinyl acetate (EVA).

In some embodiments, the implant assembly 200 can include a tab 234. The tab can be fabricated over or coupled to the central portion 214 of the implant 206. The tab 234 can facilitate in determining a central position of the implant 206 inside a patient's body. The tab 234 can be removed after placing the implant 206 inside the patient's body. In one embodiment, the tab 234 can be coupled to the sleeve 216 so as to remove the sleeve 216 along with the removal of the tab 234.

While the implant assembly 200 is shown to include the implant 206, a single sleeve 216, a set of two dilators 208 and 208b, however, the implant assembly 200 may not include one or more of these components in some other components. For example, the implant assembly 200 may not include the second dilator 208b or the implant assembly 200 may not include the sleeve 216 or the tab 234. In some other embodiments, the implant 206 may include even more components. For example, the implant assembly 200 may include a second sleeve 216b such that each of the two sleeves 216 and 216b can cover one half of the portion of the implant 206.

FIG. 3 is a perspective view of a delivery device or a delivery needle 300. The delivery needle 300 can be configured to deliver the implant assembly 200 to a target location inside the body. The delivery needle 300 includes a needle 302 and a handle 304. In some embodiments, the needle 302 can be configured to be inserted into the dilator 208 for guiding the dilator 208 and the implant 206 toward a target site inside a patient's body. In some embodiments, the needle 302 can be inserted into the lumen 230 of the dilator 208 from the proximal portion 226 of the dilator 208 and advanced into the lumen 230 till the needle 302 reaches the distal portion 228. In some embodiments, as mentioned above, the needle 302 can pierce through the closed end tip 232 of the dilator 208. In some embodiments, the needle 302 can be received into the needle slot of the dilator 208. In some embodiments, the needle 302 can be configured to be coupled to the sleeve 216 of the implant assembly 200.

The needle 302 has a proximal portion 306 and a distal portion 308. The needle 302 may include a tip portion 310 at its distal portion 308. In some embodiments, the tip portion 310 may be sharp and configured to dissect tissue layers and create a passageway within bodily tissues to deliver and place the implant 206 inside the patient's body. In some embodiments, the needle 302 can be made of stainless steel or other medical grade material. The needle 302 can define a length L6 and a width W6. In some embodiments, the needle 302 defines a curved profile and defines a radius of curvature Rl. The width W6, the length L6, and the curvature R1 of the needle 302 can depend based on the surgical requirements. In some embodiments, the width W6 and length L6 of the needle 302 can vary based on internal diameter ID2 of the lumen 230 of the dilator 208. In some embodiments, the needle 302 can be designed so as to be adapted to be used in the trans-vaginal retro pubic approach. In some embodiments, the needle 302 can be designed so as to be adapted to be used in the trans-obturator approach or vaginal pre-pubic approach. In some embodiments, the needle 302 can be designed so as to be adapted to be used in delivering the implant 206 through various other approaches inside the patient's body. In some embodiments, the delivery needle 300 can be a surgical needle 302 with a substantially small outer diameter for minimally invasive surgery.

In some embodiments, the needle 302 can be configured to frictionally engage within the lumen 230 of the dilator 208. In some embodiments, the dilator 208 can be configured to be coupled to the needle 302 such that the needle 302 is received inside the lumen 230 of the dilator 208 and fixed therein, at least temporarily. In an embodiment, the dilator 208 can be positioned over the needle 302 by sliding the dilator 208 over the needle 302, thereby forming a removable connection between the two. In some embodiments, the length L5 of the dilator 208 can be longer than the length L6 of the needle 302 such that the dilator 208 slides over the needle 302.

The delivery device 300 further includes the handle 304. In some embodiments, the handle 304 is made up of a plastic material. Exemplary plastic materials include polycarbonate, lexan, acrylonitrile butadiene styrene (ABS), and the like without limitations. The handle 304 has a proximal portion 312 and a distal portion 314 such that the distal portion 314 of the handle can be coupled to the proximal portion 306 of the needle 302. The proximal portion 312 of the handle 304 can be configured to remain outside the dilator 208 once coupled to the dilator 208 so as to remain free for manipulation by the operator.

FIG. 4A is a perspective view of an adaptor 402 in accordance with an embodiment of the invention. FIG. 4B is a cross-sectional view of the adaptor 402 of FIG. 4A. The adaptor 402 is now described in conjunction with FIGS. 2, 3, 4A, and 4B.

The adaptor 402 defines a proximal portion 404, a distal portion 406, a medial portion 414, and a lumen 430 extending from the proximal portion 404 to the distal portion 406. The adaptor 402 defines a length L7 and a width W7. In some embodiments, the width W7 of the adaptor 402 can vary from the proximal portion 404 to the distal portion 406 or can remain constant. The adaptor 402 can be configured to be coupled to the implant assembly 200. In some embodiments, the proximal portion 404 of the adaptor 402 can be coupled to the second end portion 204 of the implant assembly 200. In some embodiments, the proximal portion 404 of the adaptor 402 is configured to be coupled to the distal portion 228 of the dilator. In some embodiments, the proximal portion 404 of the adaptor 402 is configured to be coupled to the distal portion 220 of the sleeve 216. In some embodiments, the adaptor 402 can be configured to be coupled to any other component of the implant assembly 200. In some embodiments, the width W7 of the proximal portion of the adaptor 402 can be fabricated so that it is smaller than the internal diameter ID2 of the lumen 230 of the dilator 208 so that the adaptor can be inserted into the lumen 230 of the dilator 208 for coupling. In some embodiments, the width W7 can be fabricated so that it is smaller than a width of the sleeve 216 so that the adaptor can be inserted into the lumen 230 of the dilator 208 for coupling.

The lumen 430 defines an internal diameter ID3. In some embodiments as illustrated, the dimension of the internal diameter ID3 can vary from the proximal portion 404 to the distal portion 406 of the adaptor 402.

The proximal portion 404 of the adaptor 402 includes an opening 432. The opening 432 can allow a fluid to communicate between the adaptor 402 and the implant assembly 200 after the adaptor 402 has been coupled to the implant assembly 200.

The adaptor 402 may further include a locking mechanism 410 for locking the adaptor 402 to the second end portion 204 of the implant assembly 200. The locking mechanism 410 can be fabricated at the proximal portion 404 of the adaptor 402. In some embodiments, the locking mechanism 410 can include a plurality of protrusions 412. In some embodiments, the plurality of protrusions 412 can be fabricated over the proximal portion 404 of the adaptor 402. In some embodiments, the plurality of protrusions 412 can be fabricated over the proximal portion 404 and the medial portion 414 of the adaptor 402. In some embodiments as illustrated, the plurality of protrusions 412 can include barbs for snap fitting into the implant assembly 200. The barbs can have a rectangular, triangular, or oval cross-section. In some embodiments, the locking mechanism 410 can include a male coupler 416 that can be inserted into the implant assembly 200 for coupling the adaptor 402 into the implant assembly 200. In some embodiments, the male coupler 416 can be inserted into the lumen 230 of the dilator 208 at the distal portion 228 of the dilator 208 for coupling the adaptor 402 to the implant assembly 200. The adaptor 402 can similarly be configured to be coupled to the first end portion 202 of the implant assembly 200 and lock it with the use of the locking mechanism 410 at a contra lateral side.

The adaptor 402 further includes a hub portion 418. The hub portion 418 can be defined at the distal portion 406 of the adaptor 402. The hub portion 418 may include a coupler 424 and a handle 422. The coupler 424 can be provided at the hub portion 418 that is at the distal portion 406 of the adaptor 402 so as to couple the adaptor 402 to an external device such as the fluid delivery device for delivery of a fluid inside the implant assembly 200 (as illustrated and explained later by FIG.5). As shown, in some embodiments, the coupler 424 can be a female coupler. In some embodiments though as discussed in FIG. 1, the coupler can assume any other coupling design such as female coupling arrangement or a threadable engagement. The hub portion 418 defines a length L8 and a width W8. The dimensions of the length L8 and the width W8 can vary in accordance with various embodiments of the invention.

The handle 422 can be configured to facilitate application of the rotational force to the adaptor 402 with respect to the implant assembly 200 for coupling and decoupling the implant assembly 200 and the adaptor 402. For example, the handle 422 can be defined as a protruded portion around the hub portion 418 or at the central portion 414 of the adaptor 402 such that an operator can hold the handle 422 comfortably and use it as a lever for comfortably and easily applying a necessary force or torque on the proximal and distal portions 404 and 406 of the adaptor 402 to couple them to respective coupling components such as the implant assembly 200 (the dilator 208 or the sleeve 216) on one end and the fluid delivery device on the other end. The protruded portion of the handle 422 is shown to be provided on two sides of the hub portion 418 circumferentially. In other embodiments, it can be provided only on one side or entirely around the hub portion 418.

The adaptor 402 may be configured so as to facilitate application of a rotational force on such as the distal portion of the adaptor by the operator for coupling with and decoupling from the implant assembly 200. For example, the operator can apply a force or a torque along a direction A1 so as to rotate the handle 422 with respect to the adaptor 402. In some embodiments, the proximal portion 404 of the adaptor 402 can be more rigid as compared to the distal portion 406 of the adaptor 402. The more-rigid proximal portion 404 can facilitate coupling of the adaptor 402 to the implant assembly 200. In some embodiments, the adaptor 402 may include a tubular structure 408 defined at the proximal portion 404 of the adaptor 402. In some embodiments, the proximal portion 404 itself can be defined as the tubular structure 408, as shown. The tubular structure 408 defines a length L9 and a width W9 (also referred to as an outer diameter of the tubular structure 408). As shown, the locking mechanism 410 can be provided over a portion of the tubular structure 408.

In some embodiments, the tubular structure 408 can have a substantially circular cross-sectional shape. In some embodiments, the tubular structure 408 can have a square, rectangular, triangular, or any other polygonal cross sectional shape.

FIG. 4C is a perspective view of an adaptor 402B in accordance with an embodiment of the present invention.

The adaptor 402B illustrated in FIG. 4C is another embodiment of the adaptor 402. The adaptor 402B can have the tubular structure 408 extending proximally from the distal portion 406. The length L9 of the tubular structure 408 of the adaptor 402B can be relatively longer than the length L9 of the tubular structure 408 of the adaptor 402. The relatively longer length L9 of the tubular structure 408 can be inserted into the sleeve 216 of the implant assembly 200 for coupling the adaptor 402B to the implant assembly 200. Such a coupling arrangement may mitigate a requirement for the plurality of protrusions 412 for coupling with the implant assembly 200. For example, the adaptor 402B may be inserted into the sleeve 216 at the distal portion 220 of the sleeve 216 till a portion of the length L7 of the adaptor 402B is inside the sleeve 216. The insertion of a longer length of the adaptor 402B inside the sleeve 216 or the dilator 208 retains the adaptor 402B in a coupled state with the implant assembly 200 and may thus not require any other locking facility. However, in some embodiments, the plurality of protrusions 412 or the locking mechanism 410 may be present anywhere between the proximal portion 404 and the medial portion 414 of the adaptor 402B.

The adaptor 402B may further include the coupler 424. As shown, the coupler can be a thread-able arrangement. In other embodiments, the coupler can be a male or a female coupler configured to couple the adaptor to the respective fluid delivery device or any other device. In some embodiments, the adaptor may include a handle at the distal portion 406 though not shown. In some embodiments, the adaptor 402B can be a flexible or a semi flexible tube. In some embodiments, the adaptor 402 and 402B can be used interchangeably for the purpose of the invention. It must be appreciated that only two types of adaptors (402 and 402B) are discussed that may be used to be coupled to the implant assembly 200 or a portion thereof. However, several other modifications can be made to these adaptors (402 and 402B) without any limitations.

FIG. 5 is a perspective view of a medical assembly 500. In the illustrated embodiment, the medical assembly 500 includes the implant assembly 200, the delivery needle 300 and the adaptor 402. In some embodiments, the medical assembly 500 can include an implant assembly of any other type, that is, other than the implant assembly 200. In some embodiments, the medical assembly 500 can include a delivery needle of any other type, that is, other than the delivery needle 300.

The medical assembly 500 further includes a fluid delivery device 502. The fluid delivery device 502 can be filled with a fluid 504. The fluid 504 can be delivered inside a patient's body. In some embodiments, the fluid 504 can be a drug. The fluid delivery device 502 defines a proximal portion 506 and a distal portion 508 such that the proximal portion of the fluid delivery device 502 can be coupled to the adaptor 402. In some embodiments, the fluid delivery device 502 can be coupled to the distal portion 406 of the adaptor 402. In some embodiments, the fluid delivery device 502 can be configured to be coupled to the hub portion 418 of the adaptor 402. In some embodiments, the fluid delivery device 502 can be configured to be coupled to the coupler 424 of the hub portion 418. In some embodiments, the fluid delivery device 502 can include a coupler 510. In some embodiments, the coupler 510 can be a male coupler. The coupler 510 can be configured to be coupled to the coupler 424 of the hub portion 418 of the adaptor 402 for attaching the fluid delivery device 502 to the adaptor 402. In some embodiments, the fluid delivery device 502 may be configured to deliver the fluid 504 through the inlet port 222 of the implant assembly 200.

In some embodiments, the adaptor 402 may perform as a fluid injector and may directly be configured to deliver the fluid 504 to the body tissues proximate the implant site without requiring any separate fluid injection or delivery device similar to the fluid delivery device 502. For example, the fluid delivery device 502 can be integrated with the adaptor 402 and fabricated to function as a single unit.

In some embodiments, the fluid delivery device 502 can include a plunger or a syringe 512. A distal portion 516 of the plunger or syringe 512 can be manipulated by the operator. In some embodiments, the plunger or syringe 512 can be configured to be coupled to the distal portion 406 of the adaptor 402 and configured to deliver the fluid 504 through the lumen 430 to the inlet port 222 of the implant assembly 200. The inlet port 222 can deliver the fluid 504 to the patient's body proximate the delivery site through the outlet port 224. The plunger or syringe 512 can be configured as or include a fluid pump, nozzle, plunger, valve, or similar devices that are configured to draw the fluid in by for example converting pressure energy into velocity.

The fluid delivery device 502 can be configured for fluid delivery to a target site proximate the implant assembly 200 in the body of a patient such that a direction of the delivery of the fluid is different than a direction of the delivery of the implant assembly 200 inside the patient's body. For example, the implant assembly 200 is inserted inside the patient's body from the vaginal incision and moved towards the abdomen of the patient. The abdomen may have the abdominal incision over it. The implant assembly 200 may be advanced from the vaginal incision toward the abdominal incision in a direction B1. A portion of the implant assembly 200 may extend out of or be proximate to the abdominal incision after the implant assembly 200 reaches the abdomen of the patient. In some embodiments, the dilator 208 of the implant assembly 200 may extend out of or reach proximate to the abdominal incision. In some embodiments, the sleeve 216 of the implant assembly 200 may extend out of or reach proximate to the abdominal incision. In some embodiments, the proximal portion 404 of the adaptor 402 may be coupled to the portion of the implant assembly 200 that protrudes out through or reaches proximate to the second incision. The fluid delivery device 502 can be coupled to the distal portion 406 of the adaptor 402, thereby establishing a fluid communication from the fluid delivery device 502 to the implant assembly 200. The fluid delivery device 502 can be configured for injecting the fluid 504 into the implant assembly 200. As the fluid delivery device 502 is coupled to the adaptor 402 proximate the second incision, the fluid 504, injected through the fluid delivery device 502, would travel along a direction B2 from the abdominal incision to the vaginal incision and therefore, the directions B1 and B2 would be substantially different from one another. In some embodiments, the second incision can be any other incision other than the abdominal incision such as a groin incision. In such embodiments, the implant assembly 200 can be moved from the vaginal incision to the groin incision or any other incision provided and configured as the second incision. In some embodiments, the directions B1 and B2 can be opposite to each other. In some embodiments, the fluid 504 injected through the adaptor 402 travels from the second incision to the first incision along a substantially curved path inside the patient's body. Similarly, the implant assembly 102 may be delivered from the first incision to the second incision along the curved path. In such cases, the direction B2 that the fluid 504 traces along the curved path during injection of the fluid 504 is different from the direction B1 of delivery of the implant assembly 200 along the curved path.

The implant assembly 200 can be pulled along the direction B1 as the fluid 504 is injected along the direction B2 so as to place the implant 206 at a delivery site inside the patient's body. For example, the first end portion 210 of the implant 206 can be pulled along the direction B1 as the fluid 504 is injected along the direction B2 so as to place a portion of the implant 206 at a delivery site inside the patient's body. Later, the second end portion 212 of the implant 206 can be pulled along the direction B1 as the fluid 504 is injected along the direction B2 so as to place the other portion of the implant 206 at a delivery site inside the patient's body. This pulling of contra lateral sides of the implant 206 and simultaneously injecting the fluid 504 into each of the contra lateral sides places the implant 206 at a correct anatomical position and delivers the fluid 504 at the delivery site as well.

In some embodiments, an operator can aspirate along the inlet port 222 so as to determine presence of any blood vessels that can be present along a track of the implant assembly 200. It would be advantageous, as provided in some embodiments, to have a single outlet port such as the outlet port 224 for the purpose of fluid delivery through the medical assembly 500 and to determine specific locations where blood vessels are present. However, multiple outlet ports can still be provided in some embodiments. In case a blood vessel is detected along the track of the implant assembly 200, the portion of the implant assembly 200 that protrudes out through the second incision can be pulled along the direction B1 so as to reposition the implant 206.

As the portion of the implant assembly 200 protruding out of the patient's body is pulled along the direction B1, the outlet port 224 that is fabricated over the implant assembly 200 may also move along the direction B1. This may lead to a change in location of the outlet port 224 that is the location where the fluid 504 is delivered with respect to the bodily tissues. Therefore an operator may elect to inject a first fluid in a first portion of the track of the implant 206 and a second fluid in a second portion of the track of the implant 206 by pulling the implant assembly 200 and changing the location of the outlet port 224. After the implant 206 is placed and the fluid 504 has been delivered, along the track of the implant 206, the portion of the implant assembly 200 that protrudes out through the second incision can be pulled out of the patients' body so as to decouple the sleeve 216 or the dilator 208 from the implant assembly 200.

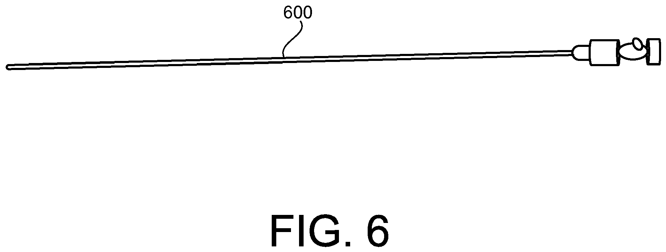

FIG. 6 is a perspective view of an exemplary spinal needle 600. The spinal needle 600 can be used for delivering drugs inside a patient's body before or during a process of treatment of pelvic floor disorder. In some embodiments, the spinal needle 600 can be used for giving anesthesia before the treatment procedure.

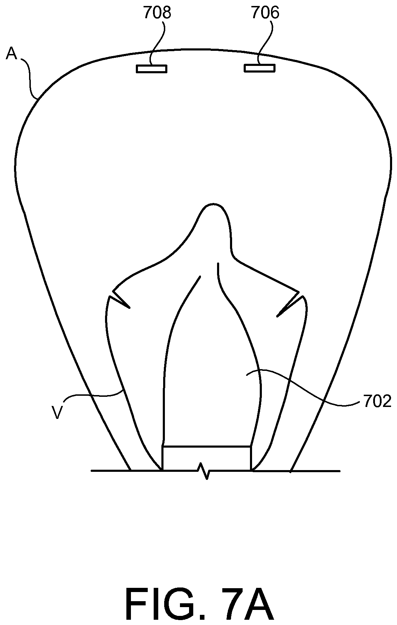

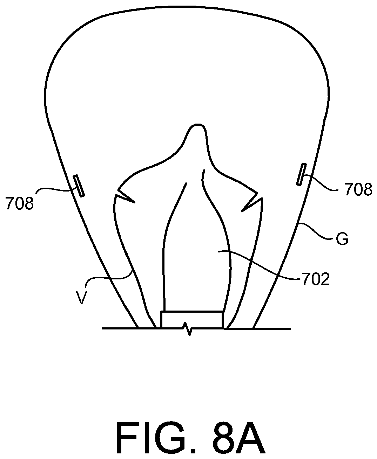

FIGS. 7A-7E illustrate a method 900 while the adaptor 402 is coupled to the dilator 208 of the implant assembly 200. In some cases, as illustrated and described later by FIGS. 8A-8C, the adaptor 402 can be coupled to the sleeve 216 of the implant assembly.

The method 900 may employ an implant assembly similar to the implant assembly 200 that can be coupled to a delivery needle similar to the delivery needle 300. The coupling can be done by inserting the distal portion 308 of the needle 302 into the lumen 230 of the dilator 208 from the proximal portion 226 of the dilator 208. The needle 302 is advanced into the lumen 230 of the dilator 208 till it reaches the distal portion 228 of the dilator 208. In some embodiments, as mentioned above, the needle 302 can pierce through the closed end tip 232 of the dilator 208. A first incision is made over a patient's body. In some embodiments, the first incision can be the vaginal incision 702 at the vagina V of the patient. A second incision is made on the patient's body at location different than the first incision. In some embodiments, the second incision can be the first abdominal incision 704 or the second abdominal incision 706 over the abdomen A of the patient as illustrated in FIG. 7A. In some embodiments, the second incision can be the groin incision 708 at the groin G of the patient. One or more than one such abdominal or groin incisions can be made.

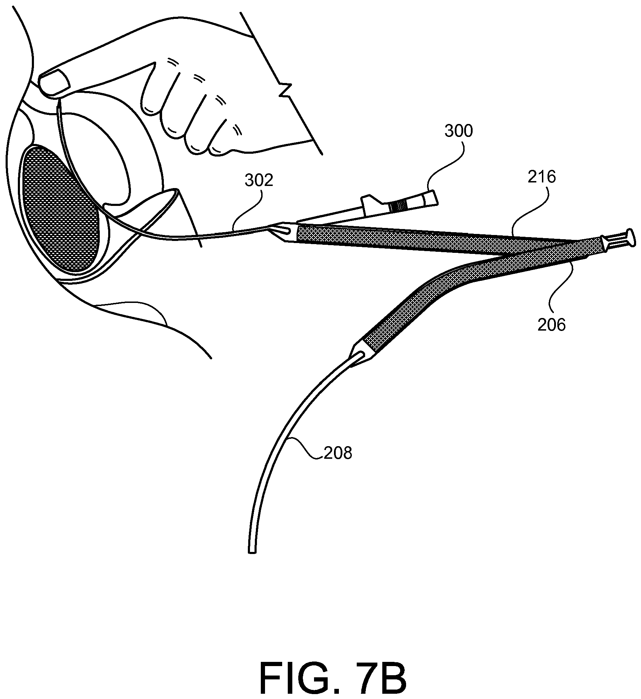

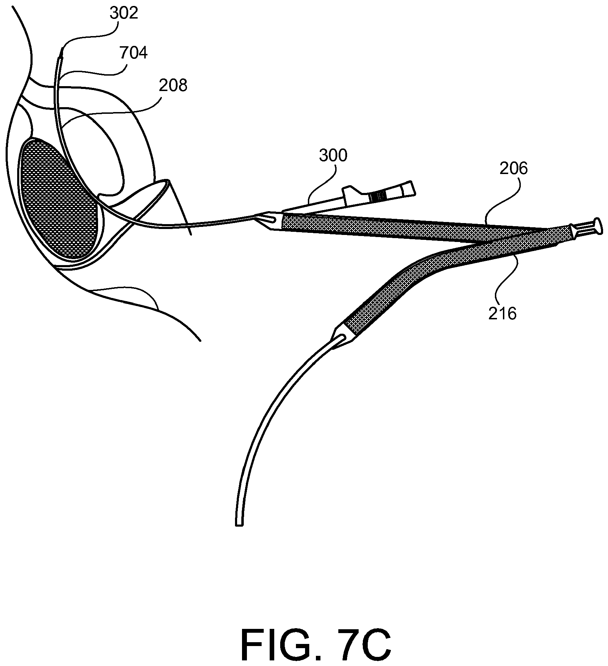

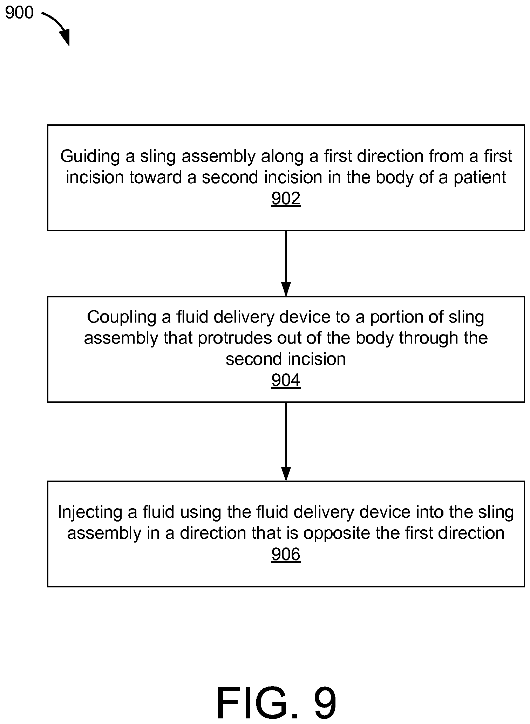

Once the incisions are made and the delivery device 300 is coupled to the implant assembly 200 for delivery inside the body, the implant assembly 200 is guided from the first incision to the second incision at step 902 of the method 900, as illustrated in FIG. 7B and 9. For example, the implant assembly 200 is inserted inside the patient's body from the vaginal incision 702 and moved towards the second incision such as the abdomen A of the patient's body where the second incision lies. The second incision can be the first abdominal incision 704 (referred to as the abdominal incision 704 hereafter). The implant assembly may be advanced from the vaginal incision toward the second incision in a direction C1. The dilator 208 of the implant assembly 200 creates a passageway for the implant assembly 200 inside the patient's body. The delivery needle 300 imparts rigidity and shape to the flexible dilator 208 for moving through the bodily tissues. The handle 304 of the delivery needle can be held by the operator and manipulated to maneuver the dilator 208 and the needle 302 into the bodily tissues. The implant assembly 200 and the delivery needle 300 can be advanced inside the patient's body till the second end portion 204 of the implant assembly 200 extends out of the patient's body, as illustrated in FIG. 7C. A portion of the implant assembly 200 such as the dilator 208, as shown, may extend out of the abdominal incision 704 or reach proximate the abdominal incision 704 upon insertion of the dilator 208 and the implant assembly 200 in the patient's body. The delivery needle 300 is pulled back and out of the patient's body from the vaginal incision 702, while the portion of the implant assembly 200, which has extended out of the patient's body through the abdominal incision 704, is left at least partially outside the patient's body.

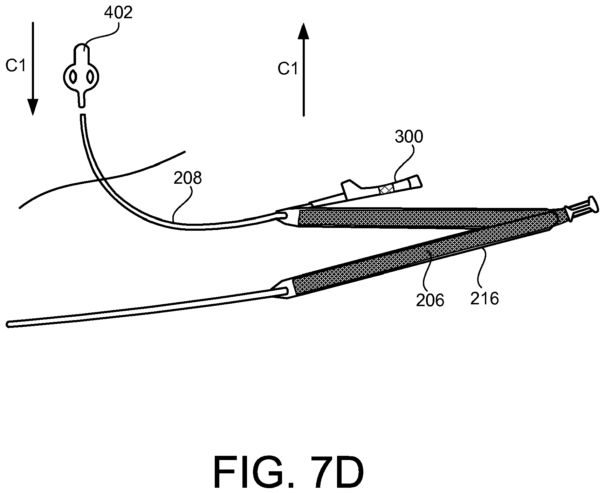

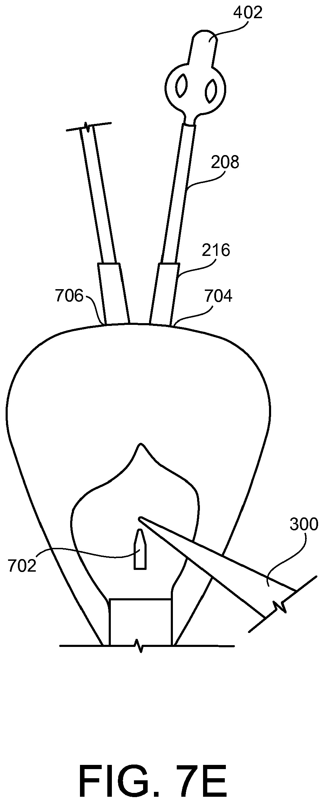

The method 900 further includes coupling an adaptor such as the adaptor 402 to the portion of the implant assembly 200 that protrudes out through the second incision 704, 706, and 708 at step 904. In some embodiments, the proximal portion 404 of the adaptor 402 can be coupled to the implant assembly 200. The coupling is done such that at least a portion of the adaptor remains outside of the body of the patient. In some embodiments, the step 904 includes coupling the proximal portion 404 of the adaptor 402, as illustrated in FIGS., 4A-4B to the dilator 208 of the implant assembly 200 that protrudes out through the abdominal incision 704. In other embodiments, other types of adaptors such as the adaptor 402B (as shown in FIG. 4C) can be coupled to the dilator 208. In such embodiments, the proximal portion 404 of the adaptor 402 or 402B can be coupled to the distal portion 228 of the dilator 208 of the implant assembly 200 by inserting the tubular structure 408 of the adaptor 402 or 402B or the proximal portion 404 of the adaptor 402, as illustrated in FIGS. 7D and 7E, into the lumen 230 of the dilator 208 or over the dilator 208.

In some embodiments the fluid 504 can be delivered through the lumen 430 of the adaptor 402 and toward the outlet port 224 in the dilator 208. In some embodiments, the method can include coupling the distal portion 406 of the adaptor 402 to the fluid delivery device 502, as described by FIG. 5, thereby establishing a fluid communication from the fluid delivery device 502 and through the lumen 430 of the adaptor 402 and to the implant assembly 200. In some embodiments, the adaptor 402 and the fluid delivery device 502 can be integral components of a single device and the fluid 504 can be delivered directly through the adaptor 402.

The method 900 further includes injecting the fluid 504 using such as the adaptor 402 into the implant assembly in a direction that is different from the first direction 702 at step 906. In some embodiments, the fluid 504 may be injected by using the fluid delivery device 502. In some embodiments, the fluid 504 may be injected by using the fluid delivery device 502 and the adaptor 402. In some embodiments, the fluid 504 may be injected directly through the adaptor 402 only. The adaptor 402 can be configured for fluid delivery to a target site proximate the implant assembly 200 in a body such as along a direction C2, as described by FIG. 5. As the adaptor 402 and the fluid delivery device 502 are coupled to the implant assembly 200 proximate the abdominal incision 704, the fluid 504 injected through the adaptor 402 or the fluid delivery device 502 would travel along the direction C2 from the abdominal incision 704 to the vaginal incision 702 or to the implant 206 and therefore, the direction C1 and C2 would be different from one other.

The step 906 may include injecting the fluid 504 through the adaptor 402 into the inlet port 222 of the implant assembly 200. The inlet port 222 is in fluid communication with the outlet port 224 that may be proximate the delivery site inside the patient's body. The fluid from the inlet port 222 can reach the outlet port 224 and the fluid 504 can thus be delivered to the bodily tissues proximate the outlet port 224, through the outlet port 224. In some embodiments, the outlet port 224 can be fabricated on the distal portion 220 of the sleeve 216 or the second end portion 204 of the implant assembly 200 or the proximal portion 226 of the dilator 208. In some embodiments, the directions C1 and C2 can be opposite to each other. In some embodiments, the fluid 504 injected through the adaptor 402 travels from the second incision to the first incision along a substantially curved path inside the patient's body. Similarly, the implant assembly 102 may be delivered from the first incision to the second incision along the curved path. In such cases, the direction C2 that the fluid 504 traces along the curved path during injection of the fluid 504 is different from the direction C1 of delivery of the implant assembly 200 along the curved path.

The method 900 further includes pulling the implant assembly 200 along the direction C1 as the fluid 504 is injected along the direction C2 so as to place the implant 206 at the delivery site inside the patient's body. In some embodiments, an operator can aspirate along the inlet port 222 so as to determine presence of any blood vessels along a track of the implant assembly 200. In some embodiments, a single outlet port similar to the outlet port 224 can be provided over a portion of the implant assembly 200 such as the dilator 208 or the sleeve 216 or the implant 206. The single outlet port 224 can advantageous as it can be used to retrieve information regarding presence of blood vessels (if any) along with the location of the blood vessel. However, use of multiple ports may though be used to retrieve information about the blood vessel but many not accurately indicate respective location of the blood vessel. In some embodiments, though multiple outlet ports can be provided without limitations.

In some embodiments, the method 900 further includes repositioning the implant assembly 200 to a new location by pulling the portion that protrudes out through the second incision upon determination of the blood vessel. As the implant assembly 200 is inserted along the direction C1, therefore, pulling the implant assembly 200 along the direction C1 can be more efficient and effective for repositioning as compared to pulling back the implant assembly 200 from the first incision along the direction C2 and pushing it again inside the patient's body. As the outlet port 224 changes its location inside the body, during movement of the implant assembly 200 such as during pulling or pushing of the portion that extends out of the second incision 704 along the direction C1 while injecting and aspirating the fluid 504 along the track of the implant assembly 200, the operator may elect to inject a first fluid such as the fluid 504 in a first portion of the track of the implant 206 and a second fluid such as a fluid different from the first fluid 504 in a second portion of the track of the implant 206.

The above description provides a discussion about an arrangement and a procedure for inserting, delivering, coupling, protruding out, pulling and placing of the first end portion 210 of the implant 206 of the implant assembly 200 for the purpose of exemplary discussion and description. In a similar manner, the second end portion 212 of the implant 206 of the implant assembly 200 may also be inserted, delivered and placed at a contra lateral side using the vaginal incision 702 and the second abdominal region 706.

After the implant 206 is placed and the fluid 504 has been delivered along the track of the implant 206, the portion of the implant assembly 200 that protrudes out through the second incision 704 can be pulled out of the patient's body so as to decouple the sleeve 216 or the dilator 208 from the implant assembly 200. In some embodiments, the method 900 further includes pulling a portion (such as a dilator, sleeve, or any other component of the implant assembly) of the implant assembly 200 out of the body of the patient through the second incision 704 after placement of the implant 206. The dilator 208 of the implant assembly 200 can be pulled along the direction C1 to remove the dilator 208 and the sleeve 216 that is coupled to the dilator after placing the implant 206 at the target site. In some embodiments, the operator can remove the dilator 208 or the sleeve 216 from the implant assembly 200 along the direction C1 and can inject the fluid 504 into the implant assembly 200 along the direction C2 simultaneously. For example, the first end portion 210 of the implant 206 can be pulled along the direction C1 from the first abdominal incision 704 as the fluid 504 is injected along the direction C2 so as to place a portion of the implant 206 at a delivery site inside the patient's body. Later, the second end portion 212 of the implant 206 can be pulled along the direction C1 from the second abdominal incision 706 as the fluid 504 is injected along the direction C2 so as to place the other portion of the implant 206 at a delivery site inside the patient's body. This pulling of contra lateral sides of the implant 206 and simultaneously injecting the fluid 504 into each of the contra lateral sides places the implant 206 at a correct anatomical position and delivers the fluid 504 at the delivery site as well.

The FIGS. 7A-7E describe the method 900 while the adaptor 402 is coupled to the dilator 208 of the implant assembly. The method 900 can be carried out with the adaptor 402 coupled to the sleeve 216, as described by FIGS 8A-8C (below are referred to in conjunction with FIGS. 2-6).

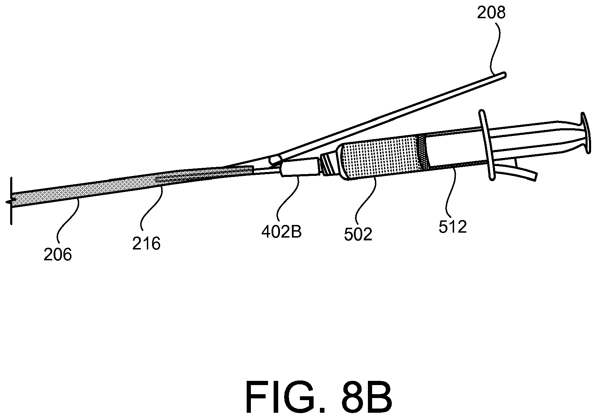

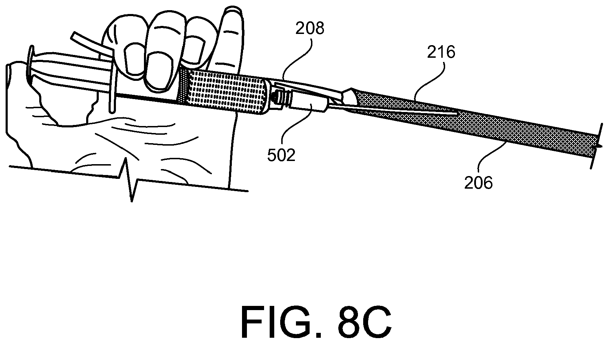

FIG. 8A is a schematic view of the first incision 702 and the groin incision 708 placed over a patient's body before insertion of the implant assembly of FIG. 2 inside the body, in accordance with an embodiment of the invention. FIG. 8B is a perspective view of the adaptor 402B and the fluid delivery device 502 coupled to the sleeve 216 of the implant assembly 200, in accordance with an embodiment of the invention. FIG. 8C is a perspective view of injecting the fluid 504 into the implant assembly 200 while the fluid delivery device 502 is coupled to a sleeve 216 of the implant assembly 200. In some embodiment, the second incision can be the groin incision 708, particularly in cases where the implant 206 is placed in an obturator foramen region of a patient's body. In such cases, the implant assembly 200 can be moved from the vaginal incision 702 to the groin incision 708. The adaptor 402B can be coupled to the sleeve 216 of the implant assembly 200 and the fluid delivery device 502 or the adaptor can inject the fluid 504 directly into the sleeve 216 of the implant assembly 200. In some embodiments, a portion of the sleeve 216 may be proximate the groin incision 704. In such cases, the adaptor 402 can be coupled to the sleeve 216 of the implant assembly 200, as illustrated in FIG. 8A. The adaptor 402B, as illustrated in FIG. 4C, can be used for the purpose of coupling the fluid delivery device 502 to the sleeve 216, in some embodiments. The adaptor 402B can have the length L7 which can be relatively longer than the adaptor 402. For example, the adaptor 402 may be inserted into the distal portion 220 of the sleeve 216 till a portion of the length L7 of the adaptor 402B is inside the sleeve 216. In some embodiments, the portion of the length L7 inside the sleeve 216 can be less than the length L4 of the sleeve 216. The overlap or insertion of the adaptor 402 with the sleeve 216, for a relatively longer length may ensure appropriate and proper coupling. The coupler 424 at the distal portion 406 of the adaptor 402B can be used for coupling the adaptor 402B to the fluid delivery device 502, in some embodiments. The outlet port 224 can be fabricated over the sleeve 216 and therefore, the length L4 of the sleeve 216 can be used for channeling the fluid 504 into the implant assembly 200 while moving the outlet port 224.

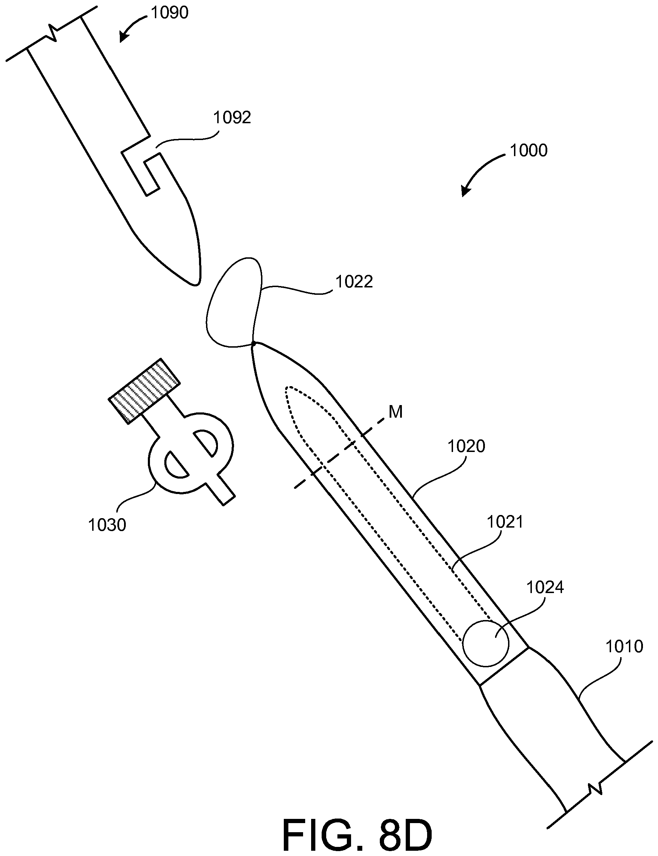

FIG. 8D illustrates an end portion of an implant assembly 1000. The implant assembly 1000 includes an implant member (not illustrated), a sleeve member 1010, and a dilator 1020. The dilatory 1020 is defines a lumen 1021 and is operatively coupled to the sleeve member 1010. The sleeve member 1010 is operatively coupled to the implant member. In some embodiments, the sleeve member 1010 defines a lumen or a cavity and at least a portion of the implant member is disposed within the lumen or cavity of the sleeve member 1010. Similar to the above-described embodiments, the opposite end portion (not illustrated) of the implant assembly 1000 may include a similar arrangement of sleeve member and dilator.

In the illustrated embodiment, an association loop 1022 is coupled to a distal end portion of the dilator 1020. The association loop 1022 may be configured to engage a needle or delivery tool 1090. For example, the needle or delivery tool 1090 may include or define a slot 1092. The slot may be configured to receive the association loop 1022 to operatively couple the association loop 1022, and thus, the implant assembly 1000, to the needle or delivery tool 1090. Accordingly, the needle or delivery tool 1090 may be placed into the body of a patient. For example, the needle or delivery tool 1090 may be placed such that it extends from a skin incision (an abdominal skin incision) to a vaginal incision. The needle or delivery tool 1090 may then be coupled to the implant assembly 1000 via the association loop 1022. The needle or delivery tool 1090 may then be retracted to pull the implant assembly 1000 into the body of the patient.

In the illustrated embodiment, the dilator 1020 defines a lumen or cavity 1021 and has a closed distal end. The distal end portion of the dilator 1020 may be cut, for example at location M. In the illustrated embodiment, the cutting of the dilator provides access to the lumen 1021 of the dilator 1020. Such access or cut may be utilized as the inlet port. An adaptor 1030 may then be coupled to the dilator 1020 via such inlet port. For example, in some embodiments the adaptor 1030 or at least a portion of the adaptor 1030 may be inserted into the inlet port to couple (such as via a frictional coupling) the adaptor 1030 to the dilator. As discussed in the embodiments above, the adaptor may be used to inject fluid into the patient via the inlet port of the dilator 1020. For example, as described in the above embodiments, the dilator 1020 may define an outlet port 1024 that is in fluid communication with the inlet port and is configured to allow the fluid to pass from the lumen 1021 of the dilator 1020 to the body of the patient.

In some embodiments, the dilator includes a plurality of outlet ports. In some embodiments, the outlet port is located or defined by a different portion of the implant assembly 1000, such as the sleeve 1010. In some embodiments, as discussed for the above embodiments, the adaptor 1030 is configured to be coupled to a syringe to provide the fluid to the body of the patient.

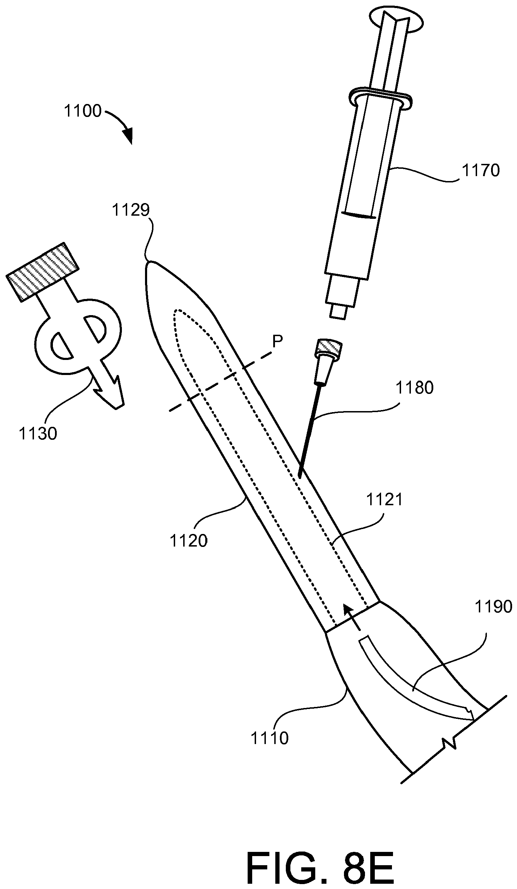

FIG. 8E illustrates an end portion of an implant assembly 1100. The implant assembly 1100 includes an implant member (not illustrated), a sleeve member 1110, and a dilator 1120. The dilatory 1120 is defines a lumen 1121 and is operatively coupled to the sleeve member 1110. The sleeve member 1110 is operatively coupled to the implant member. In some embodiments, the sleeve member 1110 defines a lumen or a cavity and at least a portion of the implant member is disposed within the lumen or cavity of the sleeve member 1110. Similar to the above-described embodiments, the opposite end portion (not illustrated) of the implant assembly 1000 may include a similar arrangement of sleeve member and dilator.

In the illustrated embodiment, the dilator 1120 includes a closed distal end portion 1129. In some embodiments, the distal end portion 1129 is tapered or includes a sharp portion or a tissue piercing portion. In the illustrated embodiment a blunt needle or delivery member 1190 may engage the dilator 1120 to push or guide the dilator 1120 and the implant assembly 1100 into the body of the patient. For example, the dilator 1120 and the implant assembly 1100 may be passed from a vaginal incision to and through a skin incision such as an abdominal skin incision. Once at least a portion of the dilator 1120 has exited the skin incision, the needle or delivery device 1190 may be removed or retraced from the body of the patient.

The dilator 1120 may then be cut, such as at location P to gain access to the lumen 1121 of the dilator. For example, an adaptor 1130 may be operatively coupled to the dilator. Alternatively an injection needle 1180 may be used to pierce a sidewall of the dilator 1120 to gain access to the lumen of the dilator 1120. The injection needle 1180 may be coupled to a syringe 1170 to deliver fluid to the body of the patient via the lumen 1121 defined by the dilator 1120. In some embodiments, the dilator 1120 defines an outlet port that is in fluid communication with the lumen 1121 to allow for delivery of the fluid to the body of the patient. In other embodiments, the dilator 1120 includes a plurality of outlet ports.

In some embodiments, a medical assembly includes an implant assembly including a proximal portion and a distal portion; and an adaptor configured to be coupled to the implant assembly at the distal portion of the implant assembly after at least a portion of the implant assembly extends through and out of the body. In some embodiments, the adaptor includes a proximal portion, a distal portion, and a lumen defined between the proximal and the distal portion; a locking mechanism for locking the adaptor to the distal portion of the implant assembly; and a hub portion defined at the distal portion of the adaptor.

In some embodiments, the implant assembly includes a dilator having a proximal portion and a distal portion. The distal portion of the dilator is configured to be coupled to the proximal portion of the adaptor. In some embodiments, the implant assembly includes an implant and a sleeve covering at least a portion of the implant. The sleeve has a proximal portion and a distal portion. The distal portion of the sleeve is configured to be coupled to the proximal portion of the adaptor.

In some embodiments, the medical assembly includes a fluid delivery device configured to be coupled to the hub portion of the adaptor and configured for fluid delivery to a target site proximate the implant assembly in a body of a patient such that a direction of the delivery of the fluid is different from a direction of the delivery of the implant assembly inside the body.

In some embodiments, the fluid delivery device includes a first coupler. The hub portion of the adaptor includes a second coupler, such that the first coupler is configured to couple with the second coupler of the adaptor for attaching the fluid delivery device to the adaptor for use in injecting the fluid in the different direction of the delivery of the implant assembly. In some embodiments, the implant assembly includes an inlet port on the distal portion of the implant assembly. The inlet port is configured for receiving the fluid from the fluid delivery device into the implant assembly. In some embodiments, the implant assembly includes an outlet port in fluid communication with the inlet port to receive the fluid from the inlet port. In some embodiments, the fluid delivery device includes a plunger.

In some embodiments, the adaptor includes a tubular structure defined at the proximal portion of the adaptor and configured to be inserted within the implant assembly; and a handle configured to facilitate rotational movement of the adaptor with respect to the implant assembly for coupling and decoupling of the adaptor. In some embodiments, the locking mechanism includes barbs for one of snap fitting and friction fitting with the implant assembly.

In some embodiments, a method for treatment of a pelvic floor disorder includes guiding an implant assembly including an implant along a first direction from a first incision toward a second incision in a body of a patient such that at least a portion of the implant assembly protrudes out from the body of the patient through the second incision; coupling a fluid delivery device to a portion of the implant assembly that protrudes out of the body through the second incision such that at least a portion of the fluid delivery device is outside of the body of the patient; and injecting a fluid using the fluid delivery device into the implant assembly in a direction that is different from the first direction.

In some embodiments, the method includes pulling the implant assembly from the second incision in the first direction so as to place the implant at a delivery site, while injecting the fluid into the implant assembly. In some embodiments, the first incision is a vaginal incision. In some embodiments, the second incision is one of an abdominal incision and a groin incision.

In some embodiments, the method includes coupling a proximal portion of an adaptor to the portion of the implant assembly that protrudes out through the second incision; and coupling a distal portion of the adaptor to the fluid delivery device. In some embodiments, he injecting of the fluid is done through the adaptor. In some embodiments, the implant assembly includes a dilator such that the adaptor is coupled to a distal portion of the dilator and the fluid is injected through the dilator from the fluid delivery device.

In some embodiments, the implant assembly includes the implant and a sleeve covering at least a portion of the implant such that the adaptor is coupled to a distal portion of the sleeve and the fluid is injected through the sleeve from the fluid delivery device. In some embodiments, the implant assembly includes a port configured to be proximate the delivery site after the implant assembly is guided through bodily tissues, and the method further includes aspirating along a track of the implant assembly within the body with the use of the port for determination of presence of a blood vessel along a track of the implant assembly. In some embodiments, the method includes repositioning the implant assembly to a new location by pulling the portion that protrudes out through the second incision upon determination of the blood vessel. In some embodiments, the method includes pulling the portion of the implant assembly that protrudes out of the body of the patient through the second incision.

In some embodiments, a method for treatment of a pelvic floor disorder includes guiding an implant assembly including an implant along a first direction from a first incision toward a second incision in a body of a patient such that at least a portion of the implant assembly protrudes out from the body of the patient through the second incision; coupling a proximal portion of an adaptor to the portion of the implant assembly that protrudes out through the second incision; and injecting a fluid through a lumen of the adaptor into the implant assembly in a direction that is different from the first direction.

In some embodiments, the method includes pulling the implant assembly from the second incision in the first direction while injecting the fluid into the implant assembly so as to place the implant at a delivery site.

While the invention has been disclosed in connection with the preferred embodiments shown and described in detail, various modifications and improvements thereon will become readily apparent to those skilled in the art.

## Claims

1. A medical assembly for the treatment of a pelvic floor disorder (100; 500) comprising:
an implant assembly (102; 200) including an implant (106; 206), a proximal portion (108; 202) and a distal portion (110; 204); and
an adaptor (104; 402; 402B) configured to be coupled to the implant assembly (102; 200) at the distal portion (110; 204) of the implant assembly (102; 200) after at least a portion of the implant assembly (102; 200) extends through and out of the body, wherein the adaptor (104; 402; 402B) includes:
a proximal portion (112; 404), a distal portion (114; 406), and a lumen (128; 430) defined between the proximal (112; 404) and the distal portion (114; 406);
a locking mechanism (118; 410) for locking the adaptor (104; 402; 402B) to the distal portion (110; 204) of the implant assembly (102; 200); and
a hub portion (120; 418) defined at the distal portion (114; 406) of the adaptor (104; 402; 402B),
**characterized in that** the medical assembly (100; 500) further comprises a fluid delivery device (502) configured to be coupled to the hub portion (120; 418) of the adaptor (104; 402; 402B) and configured for fluid delivery to a target site proximate the implant assembly (102; 200) in a body of a patient such that a direction of the delivery of the fluid is different from a direction of the delivery of the implant assembly (102; 200) inside the body.

2. The medical assembly (100; 500) of claim 1, wherein the implant assembly (106; 206) includes a dilator (208, 208B) having a proximal portion (226) and a distal portion (228), the distal portion (228) of the dilator (208, 208B) is configured to be coupled to the proximal portion (112; 404) of the adaptor (104; 402; 402B).

3. The medical assembly (100; 500) of claim 1, wherein the implant assembly (102; 200) includes a sleeve (216, 216B) covering at least a portion of the implant (106; 206), the sleeve (216, 216B) having a proximal portion (218) and a distal portion (220), the distal portion (220) of the sleeve (216,216B) configured to be coupled to the proximal portion (112; 404) of the adaptor (104; 402; 402B).

4. The medical assembly (100; 500) of claim 1, wherein the fluid delivery device (502) includes a first coupler (510), the hub portion (120; 418) of the adaptor (104; 402; 402B) including a second coupler (424), such that the first coupler (510) is configured to couple with the second coupler (424) of the adaptor (104; 402; 402B) for attaching the fluid delivery device (502) to the adaptor (104; 402; 402B) for use in injecting the fluid (504) in the different direction of the delivery of the implant assembly (102; 200).

5. The medical assembly (100; 500) of claim 1, wherein the implant assembly (102; 200) includes an inlet port (222) on the distal portion (110; 204) of the implant assembly (102; 200), the inlet port (222) configured for receiving the fluid (504) from the fluid delivery device (502) into the implant assembly (102; 200).

6. The medical assembly (100; 500) of claim 5, wherein the implant assembly (102; 200) includes an outlet port (224) in fluid communication with the inlet port (222) to receive the fluid (504) from the inlet port (222).

7. The medical device (100; 500) of claim 1, wherein the fluid delivery device (502) includes a plunger (512).

8. The medical assembly (100; 500) of claim 1, wherein the adaptor (104; 402; 402B) further includes:
a tubular structure (122; 408) defined at the proximal portion (112; 404) of the adaptor (104; 402; 402B) and configured to be inserted within the implant assembly (102; 200); and
a handle (304) configured to facilitate rotational movement of the adaptor (104; 402; 402B) with respect to the implant assembly (102; 200) for coupling and decoupling of the adaptor (104; 402; 402B).

9. The medical assembly (100; 500) of claim 1, wherein the locking mechanism (118; 410) includes barbs (412) for one of snap fitting and friction fitting with the implant assembly (102; 200).

## Patentansprüche

1. Medizinische Anordnung (100; 500) für die Behandlung einer Beckenbodenerkrankung, welche aufweist:
eine Implantatanordnung (102; 200), enthaltend ein Implantat (106; 206), einen proximalen Bereich (108; 202) und einen distalen Bereich (110; 204); und
einen Adapter (104; 402; 402B), de konfiguriert ist, mit der Implantatanordnung (102; 200) an dem distalen Bereich (110; 204) der Implantatanordnung (102; 200) gekoppelt zu werden, nachdem sich zumindest ein Teil der Implantatanordnung (102; 200) durch den und aus dem Körper heraus erstreckt, wobei der Adapter (104; 402; 402B) enthält:
einen proximalen Bereich (112; 404), einen distalen Bereich (114; 406) und ein Lumen (128; 430), das zwischen dem proximalen (112; 404) und dem distalen Bereich (114; 406) definiert ist;
einen Verriegelungsmechanismus (118; 410) zum Verriegeln des Adapters (104; 402; 402B) mit dem distalen Bereich (110; 204) der Implantatanordnung (102; 200); und
einen Nabenbereich (120; 418), der an dem distalen Bereich (114; 406) des Adapters (104; 402; 402B) definiert ist,
**dadurch gekennzeichnet, dass** die medizinische Anordnung (100; 500) weiterhin eine Fluidzuführungsvorrichtung (502) aufweist, die konfiguriert ist, mit dem Nabenbereich (120; 418) des Adapters (104; 402; 402B) gekoppelt zu sein, und konfiguriert ist für eine Fluidzuführung zu einem Zielort nahe der Implantatanordnung (102; 200) in einem Körper eines Patienten derart, dass eine Richtung des Zuführung des Fluids von einer Richtung der Zuführung der Implantatanordnung (102; 200) innerhalb des Körpers verschieden ist.

2. Medizinische Anordnung (100; 500) nach Anspruch 1, bei der die Implantatanordnung (106; 206) einen Dilatator (208, 208B) mit einem proximalen Bereich (226) und einem distalen Bereich (228) enthält, wobei der distale Bereich (228) des Dilatators (208, 208B) konfiguriert ist, mit dem proximalen Bereich (112; 404) des Adapters (104; 402; 402B) gekoppelt zu werden.

3. Medizinische Anordnung (100; 500) nach Anspruch 1, bei der die Implantatanordnung (102; 200) eine Manschette (216, 216B), die zumindest einen Bereich des Implantats (106; 206) bedeckt, enthält, wobei die Manschette (216, 216B) einen proximalen Bereich (218) und einen distalen Bereich (220) hat und der distale Bereich (220) der Manschette (216, 216B) konfiguriert ist, mit dem proximalen Bereich (112; 404) des Adapters (104; 402; 402B) gekoppelt zu werden.

4. Medizinische Anordnung (100; 500) nach Anspruch 1, bei der die Fluidzuführungsvorrichtung (502) ein erstes Kupplungsstück (510) enthält, der Nabenbereich (120; 418) des Adapters (104; 402; 402B) ein zweites Kupplungsstück (424) enthält, derart, dass das erste Kupplungsstück (510) konfiguriert ist, mit dem zweiten Kupplungsstück (424) des Adapters (104; 402; 402B) gekoppelt zu werden, um die Fluidzuführungsvorrichtung (502) an dem Adapter (104; 402; 402B) anzubringen für die Verwendung beim Injizieren des Fluids (504) in der zu der Zuführung der Implantatanordnung (102; 200) verschiedenen Richtung.

5. Medizinische Anordnung (100; 500) nach Anspruch 1, bei der die Implantatanordnung (102; 200) eine Einlassöffnung (222) in dem distalen Bereich (110; 204) der Implantatanordnung (102; 200) enthält, wobei die Einlassöffnung (222) konfiguriert ist zum Aufnehmen des Fluids (504) von der Fluidzuführungsvorrichtung (502) in die Implantatanordnung (102; 200).

6. Medizinische Anordnung (100; 500) nach Anspruch 5, bei der die Implantatanordnung (102; 200) eine Auslassöffnung (224) in Fluidverbindung mit der Einlassöffnung (222) zum Empfangen des Fluids (504) von der Einlassöffnung (222) enthält.

7. Medizinische Vorrichtung (100; 500) nach Anspruch 1, bei der die Fluidzuführungsvorrichtung (502) einen Kolben (512) enthält.

8. Medizinische Vorrichtung (100; 500) nach Anspruch 1, bei der der Adapter (104; 402; 402B) weiterhin enthält:
eine rohrförmige Struktur (122; 408), die an dem proximalen Bereich (112; 404) des Adapters (104; 402; 402B) definiert und konfiguriert ist, in die Implantatanordnung (102; 200) eingesetzt zu werden; und
einen Handgriff (304), der konfiguriert ist zum Erleichtern einer Drehbewegung des Adapters (104; 402; 402B) mit Bezug auf die Implantatanordnung (102; 200) zum Koppeln und Entkoppeln des Adapters (104; 402; 402B).

9. Medizinische Vorrichtung (100; 500) nach Anspruch 1, bei der der Verriegelungsmechanismus (118; 410) Widerhaken (412) für eine Schnapppassung und Reibungspassung mit der Implantanordnung (102; 200) enthält.

## Revendications

1. Assemblage médical pour le traitement d'un trouble du plancher pelvien (100 ; 500), comprenant :
un assemblage d'implant (102 ; 200) qui inclut un implant (106; 206), une partie proximale (108; 202) et une partie distale (110 ; 204) ; et
un adaptateur (104 ; 402 ; 402B) qui est configuré de manière à être couplé à l'assemblage d'implant (102 ; 200) au niveau de la partie distale (110 ; 204) de l'assemblage d'implant (102 ; 200) après qu'au moins une partie de l'assemblage d'implant (102 ; 200) s'étend au travers et en dehors du corps, dans lequel l'adaptateur (104 ; 402 ; 402B) inclut :
une partie proximale (112 ; 404), une partie distale (114 ; 406) et une lumière (128 ; 430) qui est définie entre la partie proximale (112 ; 404) et la partie distale (114 ; 406) ;
un mécanisme de blocage (118 ; 410) pour bloquer l'adaptateur (104 ; 402 ; 402B) sur la partie distale (110 ; 204) de l'assemblage d'implant (102 ; 200) ; et
une partie de moyeu (120 ; 418) qui est définie au niveau de la partie distale (114 ; 406) de l'adaptateur (104 ; 402 ; 402B),
**caractérisé en ce que** l'assemblage médical (100 ; 500) comprend en outre un dispositif de délivrance de fluide (502) qui est configuré de manière à être couplé à la partie de moyeu (120; 418) de l'adaptateur (102; 402; 402B) et qui est configuré de manière à délivrer un fluide sur un site cible à proximité de l'assemblage d'implant (102 ; 200) à l'intérieur du corps d'un patient de telle sorte qu'une direction de la délivrance du fluide soit différente d'une direction de la délivrance de l'assemblage d'implant (102 ; 200) à l'intérieur du corps.

2. Assemblage médical (100; 500) selon la revendication 1, dans lequel l'assemblage d'implant (106; 206) inclut un dilatateur (208, 208B) qui comporte une partie proximale (226) et une partie distale (228), la partie distale (228) du dilatateur (208, 208B) est configurée de manière à être couplée à la partie proximale (112 ; 404) de l'adaptateur (104 ; 402 ; 402B).

3. Assemblage médical (100; 500) selon la revendication 1, dans lequel l'assemblage d'implant (102 ; 200) inclut une gaine (216, 216B) qui recouvre au moins une partie de l'implant (106 ; 206), la gaine (216 ; 216B) comportant une partie proximale (218) et une partie distale (220), la partie distale (220) de la gaine (216, 216B) étant configurée de manière à être couplée à la partie proximale (112 ; 404) de l'adaptateur (104; 402 ; 402B).

4. Assemblage médical (100; 500) selon la revendication 1, dans lequel le dispositif de délivrance de fluide (502) inclut un premier coupleur (510), la partie de moyeu (120 ; 418) de l'adaptateur (104 ; 402 ; 402B) incluant un second coupleur (424), de telle sorte que le premier coupleur (510) soit configuré de manière à être couplé avec le second coupleur (424) de l'adaptateur (104; 402; 402B) pour lier le dispositif de délivrance de fluide (502) à l'adaptateur (102 ; 420 ; 402B) pour une utilisation lors de l'injection du fluide (504) dans la direction différente de la délivrance de l'assemblage d'implant (102 ; 200).

5. Assemblage médical (100; 500) selon la revendication 1, dans lequel l'assemblage d'implant (102 ; 200) inclut un orifice d'entrée (222) sur la partie distale (110 ; 204) de l'assemblage d'implant (102 ; 200), l'orifice d'entrée (222) étant configuré de manière à recevoir le fluide (504) en provenance du dispositif de délivrance de fluide (502) à l'intérieur de l'assemblage d'implant (102 ; 200).

6. Assemblage médical (100 ; 500) selon la revendication 5, dans lequel l'assemblage d'implant (102 ; 200) inclut un orifice de sortie (224) en communication de fluide avec l'orifice d'entrée (222) de manière à recevoir le fluide (504) en provenance de l'orifice d'entrée (222).

7. Assemblage médical (100; 500) selon la revendication 1, dans lequel le dispositif de délivrance de fluide (502) inclut un plongeur (512).

8. Assemblage médical (100; 500) selon la revendication 1, dans lequel l'adaptateur (104 ; 402 ; 402B) inclut en outre :
une structure tubulaire (122 ; 408) qui est définie au niveau de la partie proximale (112 ; 404) de l'adaptateur (104 ; 402 ; 402B) et qui est configurée de manière à être insérée à l'intérieur de l'assemblage d'implant (102 ; 200) ; et
un manche (304) qui est configuré de manière à faciliter un déplacement en rotation de l'adaptateur (104 ; 402 ; 402B) par rapport à l'assemblage d'implant (102 ; 200) pour le couplage et le découplage de l'adaptateur (104 ; 402 ; 402B).

9. Assemblage médical (100; 500) selon la revendication 1, dans lequel le mécanisme de blocage (118 ; 410) inclut des barbes (412) pour un ajustement pris parmi un ajustement par encliquetage et un ajustement par friction avec l'assemblage d'implant (102 ; 200).
